# EUROPEAN PATENT SPECIFICATION

(11) **EP 1 607 399 B1**
(45) Date of publication and mention of the grant of the patent: **20.01.2010**
(21) Application number: 05020467.6
(22) Date of filing: 02.05.2001
(51) Int. Cl.: C07F 9/24, A61K 31/664, A61P 35/00

(54) **BIS-(N,N'-BIS-(2-Haloethyl)Amino) Phosphoramidates as antitumor agents**
Bis-(N,N'-bis-(2-haloethyl)amino)-phosphoramidate als anti-Tumormittel
Des bis-(N,N'-bis-(2-haloethyl)amino)-phosphoramidates comme des agents anti-tumoraux

(30) Priority: 02.05.2000 US 563181
(43) Date of publication of application: 21.12.2005
(62) Divisional of application: 01935025.5
(73) Proprietor: TELIK, INC., Palo Alto, CA 94304 (US)
(72) Inventor: Herr, Jason R., Voorheesville, NY 12186 (US); Meng, Fanying, San Francisco, CA 94134 (US); Lum, Robert T., Palo Alto, CA 94306 (US); Kozlowski, Michael R., Poway, CA 92064 (US); Schow, Steven R., Redwood City, CA 94065 (US); Zhichkin, Pavel, Latham, NY 12110 (US)
(74) Representative: Barz, Peter

(56) References cited:
- KROEGER H ET AL: "Wirkung carcinostatischer Verbindungen auf die Konzentration von Diphosphopyridin- nucleotid in Tumoren" ARZNEIMITTEL FORSCHUNG. DRUG RESEARCH, ECV EDITIO CANTOR VERLAG, AULENDORF, DE, vol. 9, no. 10, 1959, pages 598-600, XP002175845 ISSN: 0004-4172
- M.H.LYTTLE ET AL.: "Glutathione-S-transferase activates novel Alkylating Agents" J.MED.CHEM., vol. 37, 1994, pages 1501-1507, XP002351412
- A.SATYAM ET AL.: "Design, Synthesis, and Evaluation of Latent Alkylating Agents activated by Glutathione S-Transferase" J.MED.CHEM., vol. 39, 1996, pages 1736-1747, XP002351413

## Description

The present invention relates to chemical compounds, pharmaceutical compositions, and processes for their preparation. More specifically, the compounds possess anti-tumor activities or are capable of being modified to have anti-tumor activities. Therefore, this invention particularly relates to the use of the compounds for the treatment of tumors and, especially, for the treatment of cancer, as well as to the processes for preparation of the compounds.

Current cancer chemotherapy protocols involve administration to patients of anti-mitotic drugs such as adriamycin, vincristine, cisplatin, doxorubicin, daunomycin and methotrexate, toxins such as diphtheria toxin, pseudomonas toxin and ricin, and anti-tumor drugs such as cyclophosphamide and isophosphamide. Cyclophosphamide is one of the most widely used anti-cancer agents in the world and is administered in combination with a number of other drugs to treat a wide variety of hematologic and solid tumors. However, several features of the cyclophosphamide detract from its clinical efficacy. For example, the drug requires metabolic activation in the liver to produce metobolites that are toxic to cancer cells. The drug is specifically toxic to the urinary bladder, and it also displays the bone marrow toxicity typical of the alkylating agent class of anti-cancer drugs. Cyclophosphamide is a potent suppressor of the immune system at the doses used to treat cancer, thus decreasing the infection-fighting ability of patients already debilitated by their disease. Finally, repeated use of cyclophosphamide frequently results in the development of resistance to the drug in a patient's cancer cells, thus rendering the drug ineffective.

Phosphoramidate derivates have long been known in the literature and are well documented as alkylating reagents. Some of them have been found to be useful in the treatment of cancer; see, for instance, H. Kröger et al., Arzneimittel Forschung . Drug Research, vol.9, no. 10, 1959, pages 598-600; and M.H. Lyttle et al., J. Med. Chem., vol. 37, 1994, pages 1501-1507. For a review see, De Vita, "Principles of Cancer Therapy", pages 765-788 in Petersdorf et al., Principles of Internal Medicine, 10th ed. McGraw-Hill, N.Y., 1983.

Cyclophosphamide analogs are also well known to the literature and have been extensively derivatized (Cyclophosphamide, Merck Index, 11th Edition pages 429-430, US5190929). However, there have been relatively few references to bis-(N,N'-bis-(2-chloroethyl)amino) phosphoramidates (DE19524515, US5306727, W09306120, DE3835772, GB2207674, DE3239858, EP072531). US5556942 discloses the compound 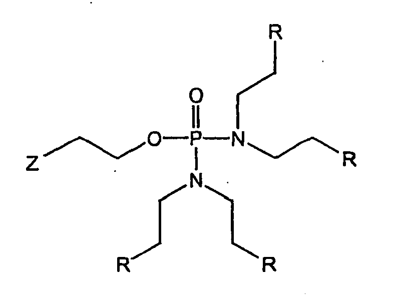 , in which Z is bromine, and R is hydrogen, or R is chlorine as a synthetic reaction intermediate.

### Summary of the Invention

One aspect of this invention relates to a new class of phosphoramidate compounds which are represented by Formula I: 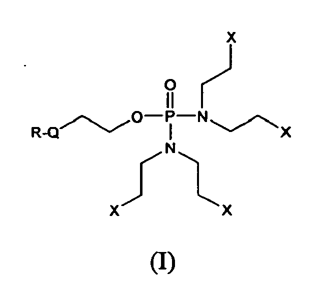 where:
X is halogen;
Q is NH; and
R is R'CO-, R'NHCO-, R'SO₂-, or R`NHSO₂-, where R' is hydrogen, C₁₋₆ alkyl, substituted C₁₋₆ alkyl, C₆₋₁₄ aryl, substituted C₆₋₁₄ aryl, C₅₋₁₄ heteroaryl, or substituted C₅₋₁₄ heteroaryl; and pharmaceutically acceptable salts thereof.

Another aspect of the invention relates to novel phosphoramidate compounds of Formula I where:
X is halogen;
Q is S; and
R is hydrogen, C₁₋₆ alkyl, substituted C₁₋₆ alkyl, C₆₋₁₄ aryl, substituted C₆₋₁₄ aryl, C₅₋₁₄ heteroaryl, or substituted C₅₋₁₄ heteroaryl, or is R'CO-, R'NHCO-, R'SO₂-, or R'NHSO₂-, where R' is hydrogen, C₁-₆ alkyl, substituted C₁₋₆ alkyl, C₆₋₁₄ aryl, substituted C₆₋₁₄ aryl, C₅₋₁₄ heteroaryl, or substituted C₅₋₁₄ heteroaryl;
said substituted C₁₋₆ alkyl being mono-, di-, or trisubstituted with C₆₋₁₄ aryl, R¹-substituted C₆₋₁₄ aryl, C₅₋₁₄ heteroaryl, nitro, cyano, halogen, -OR¹, -SR¹, -C(O)R¹, -OC(O)R¹, -C(O)OR¹, -NR¹₂, -SO₂OR¹, -OSO₂R¹, -SO₂NR¹₂, -NR¹SO₂R¹, -CONR¹₂, or -NR¹C(O)R¹, wherein each R¹ is, independently, hydrogen, C₁₋₆ alkyl, R²-substituted C₁₋₆ alkyl, C₆₋₁₄ aryl, R²-substituted C₆₋₁₄ aryl, C₅₋₁₄ heteroaryl, C₅₋₁₄ heteroaryl-C₁₋₆ alkyl, R²-substituted C₆₋₁₄ aryl-C₁₋₆ alkyl, or C₆₋₁₄ aryl-C₁₋₆ alkyl and each R² is, independently, hydroxy, halogen, C₁₋₆ alkyloxy, cyano, thio, nitro, C₁₋₆ alkyl, halo-C₁₋₆ alkyl or amino; and pharmaceutically acceptable salts thereof.
   Yet another aspect of the invention relates to a pharmaceutical composition which comprises a therapeutically effective amount of a compound of Formula I in admixture with at least one pharmaceutically acceptable carrier. Specifically, the composition is designed for treating cancer and other diseases that may benefit from an anti-mitotic agent.
   A further aspect of the invention relates to the use of a compound of formula I in the treatment of cancer and other diseases that may benefit from an anti-mitotic agent in a mammal.
   Yet another aspect of the invention relates to processes for the preparation of a compound of Formula I.

### Detailed Description of the Invention

### Definitions:

Terms used herein are based upon their recognized meanings and should be clearly understood by those skilled in the art.

The term "halogen" or "halo" means bromo, iodo, fluoro, or chloro.

The term "alkyl", as in "alkyl" or "alkyloxy", means C₁-C₆ monovalent hydrocarbyl moiety which may be linear, branched, or cyclic. This term is exemplified by radicals such as, methyl, ethyl, isopropyl, propyl, butyl, isobutyl, sec-butyl, tert-butyl, pentyl, hexyl, cyclopentyl, cyclopropylmethyl or cyclohexyl

The term "substituted alkyl" is an alkyl which is typically mono-, di-, or trisubstituted with aryl, R¹-substituted aryl, heteroaryl, nitro, cyano, halogen, -OR¹, -SR¹, -C(O)R¹, -OC(O)R¹, -C(O)OR¹, -NR'₂, -SO₂OR¹, -OSO₂R¹, -SO₂NR¹₂, -NRSO₂R¹, -CONR¹₂, or -NRC(O)R¹, wherein each R¹ is, independently, hydrogen, alkyl, R²-substituted alkyl, aryl, R²-substituted aryl, heteroaryl, heteroarylalkyl, R²-substitated arylalkyl, or arylalkyl and each R² is, independently, hydroxy, halogen, akyloxy, cyano, thio, nitro, alkyl, haloalkyl or amino. Substituted alkyls which are substituted with one to three of the substituents selected from the group consisting of cyano, halo, lower alkyloxy, thio, nitro, amino, and hydroxy are particularly preferred.

The term "haloalkyl" means a alkyl substituted with one to three halo groups, and is further exemplified by such radicals as -CF₃, -CH₂CF₃ and -CH₂CCl₃.

The term "arylalkyl" means a lower alkyl radical which is substituted with an aryl. A "substituted arylalkyl" means an arylalkyl radical having one to three substituents on the aryl portion or the alkyl portion of the radical, or both.

The term "heteroarylalkyl" means a alkyl radical which is substituted with a heteroaryl. A "substituted heteroarylaryl" means a heteroarylalkyl radical having one to three substituents on the heteroaryl portion or the alkyl portion of the radical, or both.

The term " alkyloxy" means an -OR³ radical, where R³ is alkyl.

The term "aryl", as in "aryl", "aryloxy", and "arylalkyl", means a radical derived from an aromatic hydrocarbon containing 6 to 14 ring carbon atoms, having a single ring (e.g., phenyl), or two or more condensed rings, preferably 2 to 3 condensed rings (e.g., naphthyl), or two or more aromatic rings, preferably 2 to 3 aromatic rings, which are linked by a single bond (e.g., biphenyl). The aryl radical may itself be substituted, multiply or singly, with a moiety such as an alkyl, a substituted alkyl, halogen, cyano, nitro, -SR¹, -OR¹, -C(O)R¹, -OC(O)R¹, -SO₂OR¹, -OSO₂R¹, -SO₂NR¹₂, -NRSO₂R¹, -C(O)OR¹, -NR¹₂, -CONR¹₂, or -NRC(O)R¹, wherein each R¹ is, independently, hydrogen, alkyl, R²-substituted alkyl, aryl, R²-substituted aryl, heteroaryl, heteroarylalkyl, arylalkyl, or R²-substituted arylalkyl and each R² is, independently hydroxy, halo, lower alkyloxy, cyano, thio, nitro, lower alkyl, haloalkyl or amino.

The term "substituted aryl" means an aryl substituted with one to three substituents selected from alkyl, substituted alkyl, halogen, cyano, nitro, -SR¹, -OR¹, -C(O)R¹, -OC(O)R¹, -SO₂OR¹, -OSO₂R¹, -SO₂NR¹₂, -NRSO₂R¹, -C(O)OR¹, -NR¹₂, -CONR¹₂ or -NRC(O)R¹, where each R¹ is, independently, hydrogen, alkyl, R²-substituted alkyl, aryl, R²-substituted aryl, heteroaryl, heteroarylalkyl, arylalkyl, or R²-substituted arylalkyl and each R² is, independently hydroxy, halo, alkyloxy, cyano, thio, nitro, alkyl, haloalkyl or amino. In addition, any two adjacent substituents on the aryl may optionally together form a alkylenedioxy. Particularly preferred substituents on the substituted aryl include hydroxy, halo, alkyloxy, cyano, thio, nitro, alkyl, halo-alkyl, or amino.

The term "heteroaryl", as in heteroaryl and heteroarylalkyl, means a radical derived from an aromatic hydrocarbon containing 5 to 14 ring atoms, 1 to 5 of which are hetero atoms chosen, independently, from N, O, or S, and includes monocyclic, condensed heterocyclic, and condensed carbocyclic and heterocyclic aromatic rings (e.g., thienyl, furyl, pyrrolyl, pyrimidinyl, isoxazolyl, oxazolyl, indolyl, isobenzofuranyl, purinyl, isoquinolyl, pteridinyl, imidazolyl, pyridyl, pyrazolyl, pyrazinyl, quinolyl, etc.).

The term "substituted heteroaryl" means a heteroaryl that may have from one to three substituents such as an alkyl, R¹-substituted alkyl, halo, cyano, nitro, -SR¹, -OR¹, -C(O)R¹, -OC(O)R¹, -SO₂OR¹, -OSO₂R¹, -SO₂NR¹₂, -NRSO₂R¹, -C(O)OR¹, -NR¹₂, -CONR¹₂, or -NRC(O)R¹, where each R¹ is independently hydrogen, alkyl, R²-substituted alkyl, aryl, R²-substituted aryl, heteroaryl, heteroarylalkyl, arylalkyl, or R²-substituted arylalkyl and each R² is, independently, hydroxy, halo, alkyloxy, cyano, thio, nitro, alkyl, haloalkyl, or amino. In addition, any two adjacent substituents on the heteroaryl may optionally together form a alkylenedioxy. Particularly preferred substituents on the substituted heteroaryl include hydroxy, halo, alkyloxy, cyano, thio, nitro, alkyl, haloalkyl, or amino.

The term "heterocycle", means a radical derived from an aromatic hydrocarbon containing 5 to 14 ring atoms, 1 to 5 of which are hetero atoms chosen, independently, from N, O, or S, and includes monocyclic, condensed heterocyclic, and condensed carbocyclic and heterocyclic aromatic rings (e.g., thienyl, furyl, pyrrolyl, pyrimidinyl, isoxazolyl, oxazolyl, indolyl, isobenzofuranyl, purinyl, isoquinolyl, pteridinyl, imidazolyl, pyridyl, pyrazolyl, pyrazinyl, quinolyl, etc.). Heterocycles may have from one to three substituents such as an alkyl, R¹-substituted alkyl, halo, cyano, nitro, -SR¹, -OR¹, -C(O)R¹, -OC(O)R¹, -SO₂OR¹, -OSO₂R¹, -SO₂NR¹₂, -NRSO₂R¹, -C(O)OR¹, -NR¹₂, -CONR¹₂, or -NRC(O)R¹, wherein each R¹ is independently hydrogen, alkyl, R²-substituted alkyl, aryl, R²-substituted aryl, heteroaryl, heteroarylalkyl, arylalkyl, or R²-substituted arylalkyl and each R² is, independently, hydroxy, halo, alkyloxy, cyano, thio, nitro, alkyl, haloakyl, or amino. In addition, any two adjacent substituents on the heteroaryl may optionally together form a alkylenedioxy. Particularly preferred substituents on the substituted heteroaryl include hydroxy, halo, alkyloxy, cyano, thio, nitro, alkyl, haloalkyl, or amino.

The term "ester group" means R'C(O)-O, R'CO-S-, sulfonate, or carbamate, wherein R' is hydrogen, alkyl, aryl, or heterocycle.

The term "disease", in the context of the present invention, is intended to include tumors, in particular cancer, and other diseases which may benefit from an anti-mitotic agent including but not limited to benign hyperplasia and infections by pathogenic agents such as fungal and parasitic infections.

The term "pharmaceutically acceptable salts" means salts which may be formed when acidic groups, more specifically, acidic protons present are capable of reacting with inorganic or organic bases. Acidic protons are, for example, present in the groups -OR¹, -SO₂OR¹, or -C(O)OR¹. Typically the parent compound is treated with an excess of an alkaline reagent, such as hydroxide, carbonate or alkoxide, containing an appropriate cation. Cations such as Na⁺, K⁺, Ca²⁺ and NH₄⁺ are examples of cations present in pharmaceutically acceptable salts. The Na⁺ salts are especially useful. Acceptable inorganic bases, therefore, include aluminum hydroxide, calcium hydroxide, potassium hydroxide, sodium carbonate and sodium hydroxide. Salts may also be prepared using organic bases, such as ethanolamine, diethanolamine, triethanolamine, N-methylglucamine, ethanolamine, and tromethamine. If the compounds of the invention contain a basic group, an acid addition salt may be prepared. Examples of basic groups are -NR¹₂ groups. Acid addition salts of the compounds are prepared in a standard manner in a suitable solvent from the parent compound and an excess of acid, such as hydrochloric acid, hydrobromic acid, sulfuric acid (giving the sulfate and bisulfate salts), nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, salicylic acid, p-toluenesulfonic acid, hexanoic acid, heptanoic acid, cyclopentanepropionic acid, lactic acid, o-(4-hydroxybenzoyl)benzoic acid, 1,2-ethanedisulfonic acid, 2-hydroxyethanesulfonic acid, benzenesulfonic acid, p-chlorobenzenesulfonic acid, 2-naphthalenesulfonic acid, camphorsulfonic acid, 4-methyl-bicyclo[2.2.2.]oct-2-ene-1-carboxylic acid, glucoheptonic acid, gluconic acid, 4,4'-methylenebis(3-hydroxy-2-naphthoic)acid, 3-phenylpropionic acid, trimethylacetic acid, t-butylacetic acid, laurylsulfuric acid, glucuronic acid, glutamic acid, 3-hydroxy-2-naphthoic acid, stearic acid, muconic acid and the like. The term "pharmaceutically acceptable salts" also include inner salts or Zwitterions. Zwitterions would be formed if a compound of Formula I contains both acidic protons and basic groups. "Inner salts" or "Zwitterions" can be formed by transferring a proton from the carboxyl group onto the lone pair of electrons of the nitrogen atom in the amino group.

The term "stereoisomers" means compounds that have the same sequence of covalent bonds and differ in the relative disposition of their atoms in space.

The term "therapeutically effective amount" refers to the amount which, when administered to an animal for treating a disease, is sufficient to effect such treatment for the disease.

The term "anti-mitotic" refers to a drug or agent that interferes with the division of the nucleus of a eukaryotic cell.
- The terms "treating" or "treatment" of a disease in a mammal are meant to include:
   (1) preventing the disease from occurring in a mammal which may be predisposed to the disease but does not yet experience or display symptoms of the disease,
   (2) inhibiting the disease, i.e., arresting its development, or
   (3) relieving symptoms of the disease, i.e., causing regression of the disease.

Certain compounds of the invention may contain one or more chiral centers. In such cases, all stereoisomers also fall within the scope of this invention. The compounds within the scope of the invention therefore are understood to include the individually isolated stereoisomers as well as mixtures of such stereoisomers.

### Preferred Embodiments

Within the compounds of the first aspect of the invention, certain compounds (including as pharmaceutically acceptable salts of the compounds) are preferred.
These preferences include compounds which are subject of the dependent compound claims.

### Pharmacology and Utility

The compounds of Formula I possess anti-tumor activities or are capable of being modified to have anti-tumor activities. The 2-haloethyl, more specifically, the 2-chloroethyl portion of the molecule in these compounds can act directly as an alkylator of DNA or can be transformed into an aziridinyl. The aziridinyl form is a highly active form of the molecule and is the proposed mechanism for the nitrogen mustard class of compounds. Therefore, the compounds are effective in treating tumors and other diseases which may benefit from an anti-mitotic agent, such as benign hyperplasia and infections by pathogenic agents. These compounds do not have to be metabolically activated in the liver to acquire anti-tumor activity and, therefore, do not have the same toxicity profile as cyclophosphamides.

### Administration and Pharmaceutical Compositions

The compounds of the invention may be administered in a therapeutically effective amount by any of the usual and acceptable routes to the patient being treated. Routes of administration include, but are not limited to, administration by injection, including intravenous, intraperitoneal, intramuscular, and subcutaneous injection, by transmucosal or transdermal delivery, through topical application, nasal spray, suppository and the like or may be administered orally.

The therapeutically effective amount may vary widely depending on the severity of the disease, the age and relative health of the patient, the potency of the compound used and other factors. It is usually in the range of approximately 1 milligram per Kg (mg/Kg) body weight per day to 1,000 mg/Kg body weight per day, preferably in the range of approximately 1 to 100 mg/Kg/day.

In general, compounds of the invention will be administered as pharmaceutical compositions which can take the form of tablets, pills, capsules, semisolids, powders, sustained release formulations, solutions, suspensions, elixirs, or any other appropriate composition and are comprised of, in general, a compound of formula I in combination with at least one pharmaceutically acceptable carrier. Acceptable carriers are non-toxic, aid administration, and do not adversely affect the therapeutic benefit of the compound of Formula I. Such excipient may be any solid, liquid or semisolid.

Solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk and the like. Liquid and semisolid excipients may be selected from water, ethanol, glycerol, propylene glycol and various oils, including those of petroleum, animal, vegetable or synthetic origin (e.g., peanut oil, soybean oil, mineral oil, sesame oil, etc.) Preferred liquid carriers, particularly suitable for injection solutions, include water, saline, aqueous dextrose and glycols.

The amount of a compound of Formula I in the composition may vary widely depending upon the type of composition, size of a unit dosage, kind of excipients and other factors known to those skill in the art of pharmaceutical sciences. In general, the final composition will comprise from1% w/w to 99% w/w, more preferably, 10% w/w to 90% w/w of the compound, most preferably 25% w/w to 75% w/w with the remainder being the excipient or excipients.

The pharmaceutical compositions are prepared following the conventional techniques of pharmacy.

The present invention further contemplates administration of a pharmaceutical composition of one or more compounds of Formula I to be used alone or in combination with other pharmacological agents. A preferred embodiment of the present invention includes combination therapy wherein a pharmaceutical composition of one or more compounds of Formula I are used in combination with other pharmacologically active agents selected from the group consisting of antibiotics, antineoplastic agents, tumorcidal agents, tumorstatic agents and the like. Such use of combination therapy may be readily practiced by those skilled in the art following generally accepted standards of medical care and based upon generally accepted clinical principles.

### Synthesis of Compounds of Formula I

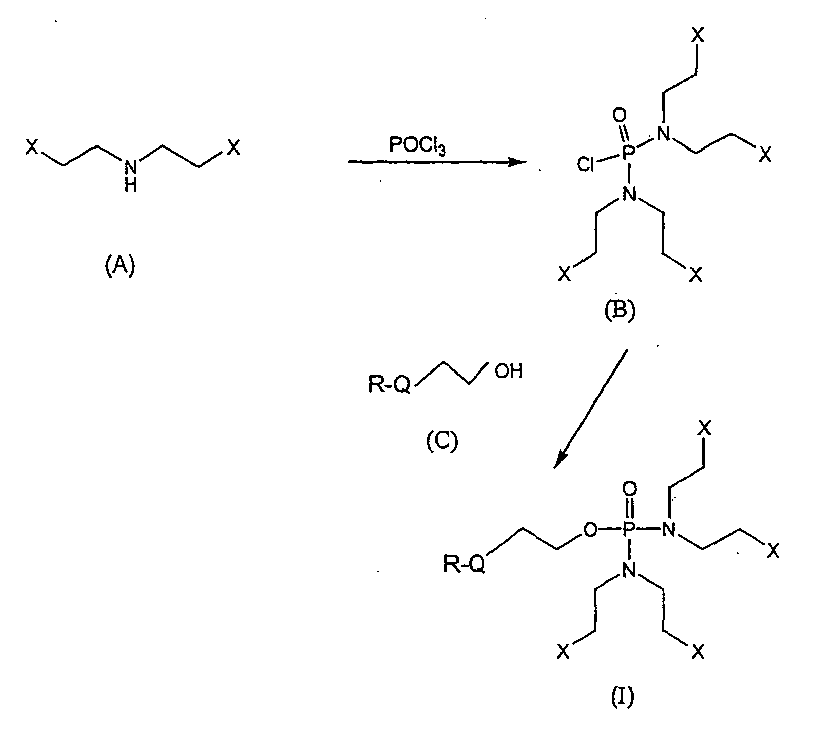

The compounds of Formula I may be prepared according to the reaction scheme depicted above and described below:
Compound **B** can be prepared by reacting phosphorous oxychloride with compound **A**, bis(2-haloethyl)amine halide. The reaction mixture is reacted in an inert solvent. Inert solvents may be aromatic hydrocarbons. A base is added to the reaction mixture, more preferably, an organic base such as organic amine, most preferably, triethylamine is added to the reaction mixture, and the reaction mixture is then stirred for an extended period of time at 0 - 50°C. The mixture, frequently a suspension, is heated to 0 - 100°C and stirred for an extended period of time. The mixture is then cooled, treated with an adsorbent, for example, charcoal, stirred at room temperature, and filtered. The solvent is then removed to produce a compound **B.**
Compound **B** is subsequently reacted with a compound C. The reaction is carried out in a polar solvent. The reaction mixture is stirred/agitated and then cooled. Alkali alkoxide is added to the mixture, which is then slowly warmed to 0 - 50°C and stirred for an extended period of time. A solution of hydrogen halide, for example, HCl, in water is introduced into the reaction mixture. The organic fraction is collected, and the aqueous fraction is extracted with a water-immiscible aprotic polar solvent (2 x 4 L). Water-immiscible aprotic polar solvents include dichloromethane, chloroform, and the like. The combined organic fractions are washed, and the solvents are removed under reduced pressure. The resulting crude product of the reaction, a compound of Formula (I), is then washed and purified by conventional means.

More specifically, compound 2 (see synthetic scheme 2, below) can be prepared by reacting phosphorous oxychloride with 1 namely, bis(2-haloethyl)amine hydrochloride. The reaction mixture in a solvent such as toluene, benzene, xylene and the like is stirred/agitated at a moderate rate (to provide a clear solution) and triethylamine is added to the reaction mixture over a period of 5-30 minutes. The reaction mixture is then stirred for an extended period of time at room temperature. To this mixture is added an additional amount of bis(2-haloethyl)amine hydrochloride and triethylamine. The resulting suspension is heated to the reflux temperature of the solvent and stirred for 16-24 hours. The mixture is then cooled to room temperature and treated with charcoal, stirred at room temperature for 2 hours and then vacuum filtered through a pad of Celite. The solvent is then removed under reduced pressure (30 mm Hg, final bath temperature at 50 °C) on a rotary evaporator to produce a compound 2.

Compound 2 thus prepared is then reacted with a compound 3. The reaction is carried out in a solvent such as tetrahydrofuran, dioxane, t-butylmethylether and the like. The reaction mixture is stirred/agitated at a moderate rate (to provide a clear red solution) and the mixture is cooled to 0°C using an ice/water bath. To this cooled solution is added potassium tert-butoxide (362 g, 3.22 moles) over 20 minutes. The reaction mixture is then slowly warmed to room temperature and stirred for an extended period of time (16-24 hours). A solution of hydrogen chloride in water is introduced into the reaction mixture at this stage. The organic fraction is collected and the aqueous fraction is extracted with ethyl acetate (2 x 4 L). The combined organic fractions are washed with saturated aqueous sodium chloride solution (1 x 4 L) and the solvents are removed under reduced pressure (30 mm Hg vacuum, final bath temperature at 50 °C) on a rotary evaporator. The resulting crude product of the reaction, a compound of Formula (I), is then washed and purified by conventional means.

In this synthesis, reactants such as phosphorous oxychloride, compounds 1 and 3 are all commercially available from sources such as Aldrich, Fluka and Alfa.

In the synthesis of certain compounds of Formula I, protective groups may be introduced and finally removed. Suitable protective groups for amino, hydroxyl, carboxyl groups are described in Greene, et al. "Protective Groups in Organic Synthesis," Second Edition, John Wiley and Sons, New York, 1991. Some derivatives require esterification of alcohols, which may be achieved by methods well known in the art (see, for example, Larock, "Comprehensive Organic Transformations", VCH Publishers, New York, 1989).

Accordingly, the process for preparing a compound of Formula I comprises one or more of the following steps:
(a) reacting a compound of Formula (2) 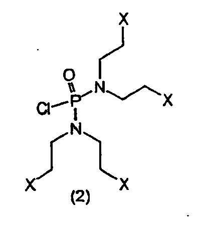 with a compound of Formula (3) 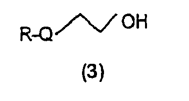 ;or,
(b) elaborating substituents of a compound of Formula I in a manner known *per* se; or
(c) converting a compound of Formula I wherein R is hydrogen and Q is oxygen into a compound of Formula I wherein R-Q together is an ester group; or
(d) reacting the free base of a compound of Formula I with an acid to give a pharmaceutically acceptable addition salt; or
(e) reacting an acid addition salt of a compound of Formula I with a base to form the corresponding free base; or
(f) converting a salt of a compound of Formula I to another pharmaceutically acceptable salt of a compound of Formula I; or
(g) resolving a racemic mixture of any proportions of a compound of Formula I to yield a stereoisomer thereof.

### Examples

The following examples are given to enable those skilled in the art to more clearly understand and to practice the present invention. They should not be considered as limiting the scope of the invention, but merely as being illustrative and representative thereof.

In the synthetic examples, thin layer chromatography was performed using 1" x 3" Analtech GF 350 silica gel plates with fluorescent indicator. Visualization of TLC plates was made by observation in iodine vapors. The proton and carbon magnetic resonance spectra were obtained on a Bruker AC 300 MHz Nuclear Magnetic Resonance spectrometer, using tetramethylsilane as an internal reference. Melting points were obtained using an electrothermal melting point apparatus and are uncorrected. Infrared spectra were obtained as KBr pellets and obtained on a Perkin-Elmer Spectrum 1000 FT-Infrared spectrophotometer. Mass spectroscopic analyses were performed on a Shimadzu QP-5000 GC/Mass Spectrometer (CI, methane) by direct injection. Thermal analyses were run on a Mettler Toledo DSC821 e Differential Scanning Calorimeter.

### Synthesis of Compounds of Formula I

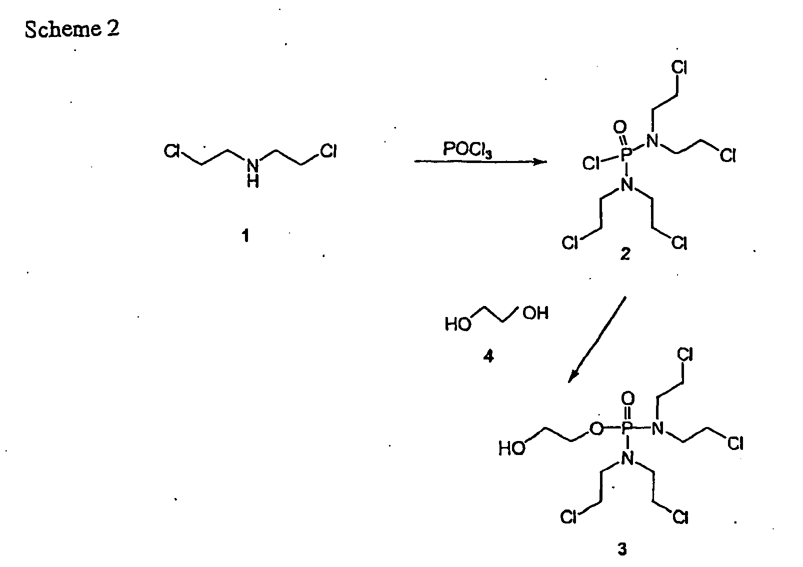

### Example 1

### Preparation of N,N,N',N'-Tetrakis(2-chloroethyl)phosphorodiamidic Chloride (2)

A 12-L, three-neck, round-bottom flask in an electric heating mantle was equipped with an overhead mechanical stirrer, a reflux condenser and a 2-L pressure-equalizing addition funnel capped with a nitrogen inlet/outlet bubbler. The flask was charged with phosphorous oxychloride (258 mL, 2.77 moles), bis(2-chloroethyl)amine hydrochloride (495 g, 2.77 moles), **1**, and toluene (5 L). The stirrer was set to agitate at a moderate rate (to provide a clear solution) and triethylamine (812 mL, 5.82 moles) was added to the reaction mixture over 10 minutes. The reaction mixture was then stirred for 26 hours at room temperature. To this mixture was charged bis(2-chloroethyl)amine hydrochloride (505 g, 2.83 moles) and triethylamine (830 mL, 6.11 moles) over 10 minutes. The tan suspension was heated to toluene reflux (110 °C) and stirred for 22 hours. The mixture was then cooled to room temperature and treated with charcoal (500 g), stirred at room temperature for 2 hours and then vacuum filtered through a pad of Celite (1 kg). The solvent was removed under reduced pressure (30 mm Hg, final bath temperature at 50 °C) on a rotary evaporator to produce *N,N,N',N'*-tetrakis(2-chloroethyl)phosphorodiamidic chloride, **2**, as a reddish-brown oil (979 g, 97% yield). TLC analysis showed one spot (*R_{f}*=0.71; ethyl acetate:hexanes 1:1). The proton NMR spectrum was consistent with commercially available material.

### Example 2

### Preparation of 2-Hydroxyethyl N,N,N',N'-Tetrakis(2-chloroethyl)-phosphorodiamidate ,3 (not a compound of the invention)

A 12-L, three-neck, round-bottom flask was equipped with an overhead mechanical stirrer, a thermometer and a nitrogen inlet/outlet bubbler. The flask was charged with *N,N,N,N*'-tetrakis(2-chloroethyl)phosphorodiamidic chloride (2) (979 g, 2.68 moles), ethylene glycol (1.5 L, 26.8 moles), 4 and tetrahydrofuran (3 L). The stirrer was set to agitate at a moderate rate (to provide a clear red solution) and the mixture was cooled to 0 °C using an ice/water bath. To this cooled solution was added potassium tert-butoxide (362 g, 3.22 moles) over 20 minutes. The reaction mixture was then slowly warmed to room temperature and stirred for a total of 19 hours. A solution of hydrogen chloride in water (8 L, 1M) was introduced into the flask over 10 minutes. The organic fraction was collected and the aqueous fraction was extracted with ethyl acetate (2 x 4 L). The combined organic fractions were washed with saturated aqueous sodium chloride solution (1 x 4 L) and the solvents were removed under reduced pressure (30 mm Hg vacuum, final bath temperature at 50 °C) on a rotary evaporator to provide a dark reddish brown oil. This crude organic product was then dissolved with warm (40 °C) methyl tert-butyl ether (2 L) and allowed to cool to room temperature, stirring for a total of 14 hours. The resulting slurry was stirred for 30 minutes at 0 °C after which the precipitate was collected by vacuum filtration and washed with cold methyl tert-butyl ether (1 L). The combined filtrates were concentrated under reduced pressure (30 mm Hg vacuum, final bath temperature at 50 °C) on a rotary evaporator to a volume of 500 mL and stirred for 30 minutes at 0 °C. The resulting precipitate was collected by vacuum filtration and washed with cold methyl tert-butyl ether (200 mL). Two crops of solid product were then combined and dried overnight (30 mm Hg, 40 °C) to produce 2-hydroxyethyl *N,N,N',N*'-tetrakis(2-chloroethyl)phosphorodiamidate, **3**, as a tan powder (526 gm, 50% yield). TLC analysis showed one spot (*R_{f}* 0.62; methanol:chloroform 1:9). Melting point: 76-78 °C; IR (KBr) 3372, 2957, 1459, 1344, 1206, 1098, 1053, 929 cm⁻¹; MS (CI, methane) m/z 391(MH⁺), 353, 339, 248, 106. A thermal analysis (DSC) showed two exothermic decomposition ranges of 164-179 and 222-231 C:

**Table 1, below, shows compounds of the invention made by a similar method**

| 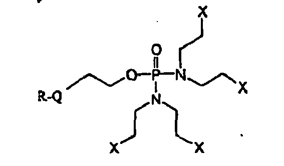 | | | |
|---|---|---|---|
| **Compound** | **R-Q** | **X** | **MW** |
| **6** | NH₂ | Cl | 389 |
| **7** | SH | Cl | 406 |

### Example 3

### Preparation of Compound 11 (Scheme 3) (not a compound of the invention)

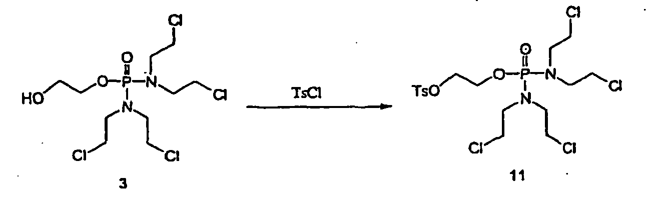

To a solution of compound 3 (0.780gm, 2 mmol) in THFM20 (1:1) and 1N NaOH (2 mL), was added a solution of p-toluene sulfonylchloride (TsCl, 0.40gm, 2.1 mmol) in THF. The reaction mixture was allowed to stir for 24h at RT. The mixture was diluted with methylene chloride, and washed with 1N HCl. The organic layer was washed with brine, dried over K₂CO₃ and concentrated to a viscous oil, compound 11 (1.04gm, 96%)

**Table 2, below, shows compounds of the invention that were prepared in a similar fashion**

| 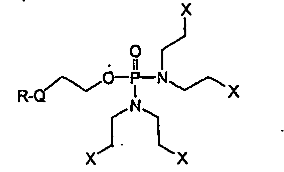 | | | |
|---|---|---|---|
| **Compound** | **R** | **Q** | **X** |
| **21** | Ac | NH | Cl |
| **22** | PhCO | NH | Cl |
| **23** | p-MePhSO₂ | NH | Cl |

### Example 4 Cell Viability

RPMI 8322 human melanoma cells (ATCC) were grown at 37 C in MEM supplemented with 10% fetal bovine serum, 1% L-glutamate, and 0.1% gentamicin. The cells were plated at a density of 2 X 10⁶ per well in 96-well microtiter plates. Twenty four hours later, the medium was replaced with the same medium to which test compounds at final concentrations of 1µM to 10mM, and 1% DMSO had been added. The cells were grown in the presence of the compound for an additional 4 days. Each concentration of the compound was tested in triplicate. Cell viability was measured using a standard assay that monitors metabolic conversion of a dye (CellTiter 96™, Promega).

| **Compound** | **µM to 50% cell viability** |
|---|---|
| **6** | >500 |
| **23** | >500 |

### Example 5 Anti-tumor Effects in Mice

Female, 7 weeks old NCr-*nu* athymic nude mice were purchased from Charles River Laboratories (Raliegh, NC) and acclimated in the laboratories one week prior to experimentation. The animals were housed in microisolator cages, at five per cage, in a 12-hour light/dark cycle. The animals received filtered sterilized water and sterile rodent food *ad libitum.* Animals were observed daily, and clinical signs such as tumor shrinkage were noted.

*Tumor Model.* Thirty- to forty mg fragments of MX-1 human mammary tumor were implanted subcutaneously in mice near the right axillary area, using a 12-gauge trocar needle and allowed to grow. Tumors were allowed to reach 75-196 mg in weight (75-196 mm³ in size) before the start of treatment.

*Drug Treatment*. Compound 3 was administered intraperitoneally for five consecutive days at dosages of 55, 75, and 100 mg/kg/dose. Compound 3 was formulated fresh daily at a concentration of 10mg/mL in 5% EtOH/45% propylene glycol/50% PBS. Dosing solutions were administered to mice within 4 hours of formulation. Compounds were administered by exact body weight, with the injection volume being 0.1mL/10g body weight.

*Tumor measurements.* The tumors were measured, and the animals were weighed twice weekly, starting with the first day of treatment. Tumor volume was determined by caliper measurements (mm) and using the formula for an ellipsoid sphere L x W²/2=mm³, where L and W refer to the larger and smaller dimensions collected at each measurement. This formula was also used to calculate tumor weight, assuming a unit density (1 mm³ = 1 mg).

Study duration was 28 days after tumor implantation. Any animal whose tumor reached 4 g in weight was sacrificed prior to study termination.

| Dose (mg/Kg) | Non-Specific Deaths | Tumor Regression | | Tumor-Free | Days to Doubling |
|---|---|---|---|---|---|
| | | Partial Regression | Complete Regression | | |
| 100 | 0/8 | 0 | 0 | 0/8 | 7.7 |
| 75 | 0/8 | 0 | 0 | 0/8 | 8.1 |
| 55 | 0/8 | 0 | 0 | 0/8 | 7.2 |
| PBS control | | | | 0/8 | 6.6 |

## Claims

1. A compound of the formula (I): 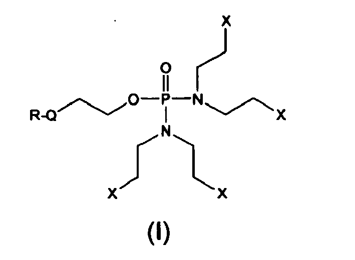 where:
X is halogen;
Q is NH; and
R is R'CO-, R'NHCO-, R'SO₂-, or R'NHSO₂-, where R' is hydrogen, C₁₋₆ alkyl, substituted C₁₋₆ alkyl, C₆₋₁₄ aryl, substituted C₆₋₁₄ aryl, C₅₋₁₄ heteroaryl, or substituted C₅₋₁₄ heteroaryl;
and pharmaceutically acceptable salts thereof.

2. The compound of claim 1 where X is chlorine or bromine.

3. The compound of claim 2 where X is chlorine.

4. The compound of claim 3 where R is R'SO₂-.

5. The compound of claim 4 where R is methanesulfonyl, para-nitrobenzenesulfonyl, para-bromobenzenesulfonyl, or para-toluenesulfonyl.

6. The compound of claim 3 where R is acetyl.

7. The compound of claim 3 where R is benzoyl.

8. A pharmaceutical composition comprising a compound of any one of claims 1-7.

9. The pharmaceutical composition of claim 8 for the treatment of tumors, in particular cancer, in a mammal.

10. A compound of any one of claims 1-7 for use in the treatment of tumors, in particular cancer, in a mammal.

11. A process for the preparation of a compound of formula (I) according to claim 1, the process comprising
(a) reacting a compound of formula (2) 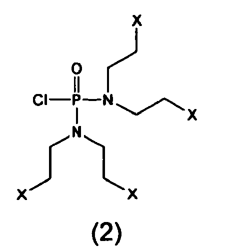 with a compound of formula (3) 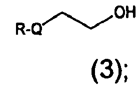 or
(b) elaborating substituents of a compound of formula I in a manner known *per se*; or
(c) reacting the free base of a compound of formula I with an acid to give a pharmaceutically acceptable acid addition salt, or
(d) reacting an acid addition salt of a compound of formula I with a base to form the corresponding free base, or
(e) converting a salt of a compound of formula I to another pharmaceutically acceptable salt of a compound of formula I; or
(f) resolving a racemic mixture of any proportions of a compound of formula I to yield a stereoisomer thereof.

12. A compound of the formula (I): 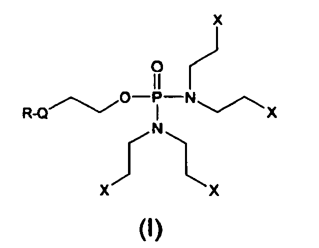 where:
X is halogen;
Q is S; and
R is hydrogen, C₁₋₆ alkyl, substituted C₁₋₆ alkyl, C₆₋₁₄ aryl, substituted C₆₋₁₄ aryl,
C₅₋₁₄ heteroaryl, or substituted C₅₋₁₄ heteroaryl, or is R'CO-, R'NHCO-, R'SO₂- or R'NHSO₂-, where R' is hydrogen, C₁₋₆ alkyl, substituted C₁₋₆ alkyl, C₆₋₁₄ aryl, substituted C₆₋₁₄ aryl, C₅₋₁₄ heteroaryl, or substituted C₅₋₁₄ heteroaryl; said substituted C₁₋₆ alkyl being mono-, di-, or trisubstituted with C₆₋₁₄ aryl, R¹-substituted C₆₋₁₄ aryl, C₅₋₁₄ heteroaryl, nitro, cyano, halogen, -OR¹, -SR¹, -C(Q)R¹, -OC(O)R¹, -C(O)OR¹, -NR¹₂, -SO₂OR¹, -OSO₂R¹, -SO₂NR¹₂, -NR¹SO₂R¹, -CONR¹₂, or -NR¹C(O)R¹, wherein each R¹ is, independently, hydrogen, C₁₋₆ alkyl,
R²-substituted C₁₋₆ alkyl, C₆₋₁₄ aryl, R²-substituted C₆₋₁₄ aryl, C₅₋₁₄ heteroaryl, C₅₋₁₄ heteroaryl-C₁₋₆ alkyl, R²-substituted C₆₋₁₄ aryl-C₁₋₆ alkyl, or C₆₋₁₄ aryl-C₁₋₆ alkyl and each R² is, independently, hydroxy, halogen, C₁₋₆ alkyloxy, cyano, thio, nitro, C₁₋₆ alkyl, halo-C₁₋₆ alkyl or amino;
and pharmaceutically acceptable salts thereof.

13. The compound of claim 12 where X is chlorine or bromine.

14. The compound of claim 13 where X is chlorine.

15. The compound of any one of claims 12-14 where R is hydrogen, C₁₋₆ alkyl, substituted C₁₋₆ alkyl, C₆₋₁₄ aryl, substituted C₆₋₁₄ aryl, C₅₋₁₄ heteroaryl, or substituted C₅₋₁₄ heteroaryl.

16. The compound of claim 14 where R is H.

17. A pharmaceutical composition comprising a compound of any one of claims 12-16.

18. The pharmaceutical composition of claim 17 for the treatment of tumors, in particular cancer, in a mammal.

19. A compound of any one of claims 12-16 for use in the treatment of tumors, in particular cancer, in a mammal.

20. A process for the preparation of a compound of formula (I) according to claim 12, the process comprising
(a) reacting a compound of formula (2) 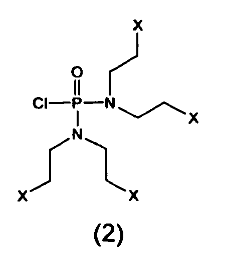 with a compound of formula (3) 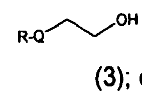 or
(b) elaborating substituents of a compound of formula I in a manner known per *se;* or
(c) reacting the free base of a compound of formula I with an acid to give a pharmaceutically acceptable acid addition salt, or
(d) reacting an acid addition salt of a compound of formula I with a base to form the corresponding free base, or
(e) converting a salt of a compound of formula I to another pharmaceutically acceptable salt of a compound of formula I; or
(f) resolving a racemic mixture of any proportions of a compound of formula I to yield a stereoisomer thereof.

## Patentansprüche

1. Verbindung der Formel (I): 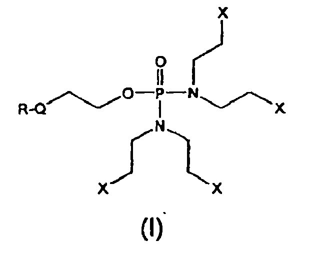 worin:
X Halogen ist;
Q NH ist; und
R R'CO-, R'NHCO-, R'SO₂- oder R'NHSO₂- ist, worin R' Wasserstoff, C₁₋₆-Alkyl, substituiertes C₁₋₆-Alkyl, C₆₋₁₄-Aryl, substituiertes C₆-₁₄-Aryl, C₅₋₁₄-Heteroaryl oder substituiertes C₅₋₁₄-Heteroaryl ist;
und pharmazeutisch verträgliche Salze davon.

2. Verbindung nach Anspruch 1, worin X Chlor oder Brom ist.

3. Verbindung nach Anspruch 2, worin X Chlor ist.

4. Verbindung nach Anspruch 3, worin R R'SO₂- ist.

5. Verbindung nach Anspruch 4, worin R Methansulfonyl, para-Nitrobenzolsulfonyl, para-Brombenzolsulfonyl oder para-Toluolsulfonyl ist.

6. Verbindung nach Anspruch 3, worin R Acetyl ist.

7. Verbindung nach Anspruch 3, worin R Benzoyl ist.

8. Pharmazeutische Zusammensetzung, umfassend eine Verbindung nach irgendeinem der Ansprüche 1 bis 7.

9. Pharmazeutische Zusammensetzung nach Anspruch 8 zur Behandlung von Tumoren, insbesondere Krebs, bei einem Säugetier.

10. Verbindung nach irgendeinem der Ansprüche 1 bis 7 zur Verwendung bei der Behandlung von Tumoren, insbesondere Krebs, bei einem Säugetier.

11. Verfahren zur Herstellung einer Verbindung der Formel (I) nach Anspruch 1, wobei das Verfahren umfasst
(a) Umsetzen einer Verbindung der Formel (2) 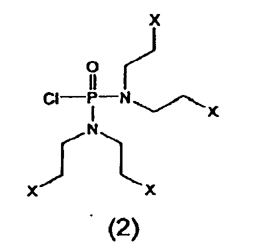 mit einer Verbindung der Formel (3) 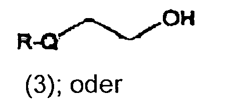
(b) Ausarbeiten von Substituenten einer Verbindung der Formel I in einer an sich bekannten Weise; oder
(c) Umsetzen der freien Base einer Verbindung der Formel I mit einer Säure, um ein pharmazeutisch verträgliches Säureadditionssalz zu ergeben, oder
(d) Umsetzen eines Säureadditionssalzes einer Verbindung der Formel I mit einer Base, um die entsprechende freie Base zu bilden, oder
(e) Umwandeln eines Salzes einer Verbindung der Formel I in ein anderes pharmazeutisch verträgliches Salz einer Verbindung der Formel I; oder
(f) Racematspaltung einer racemischen Mischung in beliebigen Verhältnissen von einer Verbindung der Formel I, um ein Stereoisomer davon zu gewinnen.

12. Verbindung der Formel (I): 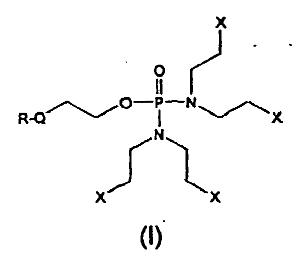 worin:
X Halogen ist;
Q S ist; und
R Wasserstoff, C₁₋₆-Alkyl, substituiertes C₁₋₆-Alkyl, C₆₋₁₄-Aryl, substituiertes C₆₋₁₄-Aryl, C₅₋₁₄-Heteroaryl oder substituiertes C₅₋₁₄-Heteroaryl ist oder R'CO-, R'NHCO-, R'SO₂- oder R'NHSO₂- ist, worin R' Wasserstoff, C₁₋₆-Alkyl, substituiertes C₁₋₆-Alkyl, C₆₋₁₄-Aryl, substituiertes C₆₋₁₄-Aryl, C₅₋₁₄-Heteroaryl oder substituiertes C₅₋₁₄-Heteroaryl ist; wobei das substituierte C₁-₆-Alkyl mono-, di- oder trisubstituiert ist mit C₆₋₁₄-Aryl, R¹-substituiertem C₆₋₁₄-Aryl, C₅₋₁₄-Heteroaryl, Nitro, Cyano, Halogen, -OR¹, -SR¹, -C(O)R¹, -OC(O)R¹, -C(O)OR¹, -NR'₂, -SO₂OR¹, -OSO₂R¹, -SO₂NR¹₂, -NR¹SO₂R¹, -CONR¹₂ oder -NR¹C(O)R¹, worin jedes R¹ unabhängig Wasserstoff, C₁₋₆-Alkyl, R²-substituiertes C₁₋₆-Alkyl, C₆₋₁₄-Aryl, R²-substituiertes C₆₋₁₄-Aryl, C₅₋₁₄-Heteroaryl, C₅₋₁₄-Heteroaryl-C₁₋₆-alkyl, R²-substituiertes C₆₋₁₄-Aryl-C₁₋₆-alkyl oder C₆₋₁₄-Aryl-C₁₋₆-alkyl ist und jedes R² unabhängig Hydroxy, Halogen, C₁₋₆-Alkyloxy, Cyano, Thio, Nitro, C₁₋₆-Alkyl, Halogen-C₁-₆-alkyl oder Amino ist;
und pharmazeutisch verträgliche Salze davon.

13. Verbindung nach Anspruch 12, worin X Chlor oder Brom ist.

14. Verbindung nach Anspruch 13, worin X Chlor ist.

15. Verbindung nach irgendeinem der Ansprüche 12 bis 14, worin R Wasserstoff, C₁₋₆-Alkyl, substituiertes C₁₋₆-Alkyl, C₆₋₁₄-Aryl, substituiertes C₆₋₁₄-Aryl, C₅₋₁₄-Heteroaryl oder substituiertes C₅₋₁₄-Heteroaryl ist.

16. Verbindung nach Anspruch 14, worin R H ist.

17. Pharmazeutische Zusammensetzung, umfassend eine Verbindung nach irgendeinem der Ansprüche 12 bis 16.

18. Pharmazeutische Zusammensetzung nach Anspruch 17 zur Behandlung von Tumoren, insbesondere Krebs, bei einem Säugetier.

19. Verbindung nach irgendeinem der Ansprüche 12 bis 16 zur Verwendung bei der Behandlung von Tumoren, insbesondere Krebs, bei einem Säugetier.

20. Verfahren zur Herstellung einer Verbindung der Formel (I) nach Anspruch 12, wobei das Verfahren umfasst
(a) Umsetzen einer Verbindung der Formel (2) 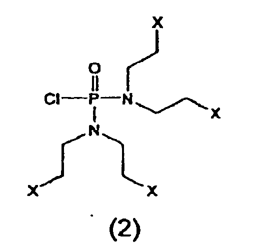 mit einer Verbindung der Formel (3) 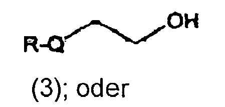
(b) Ausarbeiten von Substituenten einer Verbindung der Formel I in einer an sich bekannten Weise; oder
(c) Umsetzen der freien Base einer Verbindung der Formel I mit einer Säure, um ein pharmazeutisch verträgliches Säureadditionssalz zu ergeben, oder
(d) Umsetzen eines Säureadditionssalzes einer Verbindung der Formel I mit einer Base, um die entsprechende freie Base zu bilden, oder
(e) Umwandeln eines Salzes einer Verbindung der Formel I in ein anderes pharmazeutisch verträgliches Salz einer Verbindung der Formel I; oder
(f) Racematspaltung einer racemischen Mischung in beliebigen Verhältnissen von einer Verbindung der Formel I, um ein Stereoisomer davon zu gewinnen.

## Revendications

1. Composé de formule (I) : 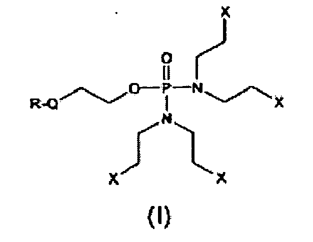
dans laquelle :
X représente un atome d'halogène ;
Q représente un groupe NH ; et
R représente un groupe R'CO-, R'NHCO-, R'SO₂ ou R'NHSO₂, dans lequel R' représente un atome d'hydrogène, un groupe alkyle en C₁ à C₆, alkyle en C₁ à C₆ substitué, aryle en C₆ à C₁₄, aryle en C₆ à C₁₄ substitué, hétéroaryle en C₅ à C₁₄ ou hétéroaryle en C₅ à C₁₄ substitué ;
et ses sels pharmaceutiquement acceptables.

2. Composé suivant la revendication 1, dans lequel X représente un atome de chlore ou de brome.

3. Composé suivant la revendication 2, dans lequel X représente un atome de chlore.

4. Composé suivant la revendication 3, dans lequel R représente un groupe R'SO₂-.

5. Composé suivant la revendication 4, dans lequel R représente un groupe méthanesulfonyle, para-nitrobenzène-sulfonyle, para-bromobenzènesulfonyle ou para-toluène-sulfonyle.

6. Composé suivant la revendication 3, dans lequel R représente un groupe acétyle.

7. Composé suivant la revendication 3, dans lequel R représente un groupe benzoyle.

8. Composition pharmaceutique comprenant un composé de l'une quelconque des revendications 1 à 7.

9. Composition pharmaceutique suivant la revendication 8, pour le traitement de tumeurs, en particulier d'un cancer, chez un mammifère.

10. Composé suivant l'une quelconque des revendications 1 à 7, destiné à être utilisé dans le traitement de tumeurs, en particulier du cancer, chez un mammifère.

11. Procédé pour la préparation d'un composé de formule (I) suivant la revendication 1, le procédé comprenant
(a) la réaction d'un composé de formule (2) 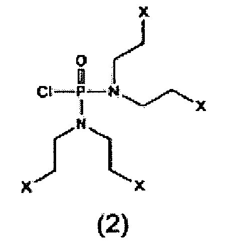 avec un composé de formule (3) 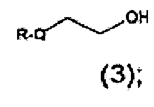 ou
(b) la transformation de substituants d'un composé de formule I d'une manière en elle-même connue ; ou
(c) la réaction de la base libre d'un composé de formule I avec un acide pour obtenir un sel d'addition d'acide pharmaceutiquement acceptable, ou
(d) la réaction d'un sel d'addition d'acide d'un composé de formule I avec une base pour obtenir la base libre correspondante, ou
(e) la conversion d'un sel d'un composé de formule I en un autre sel pharmaceutiquement acceptable en un composé de formule I ; ou
(f) la résolution d'un mélange racémique en n'importe quelles proportions d'un composé de formule I pour obtenir un de ses stéréoisomères.

12. Composé de formule (I) : 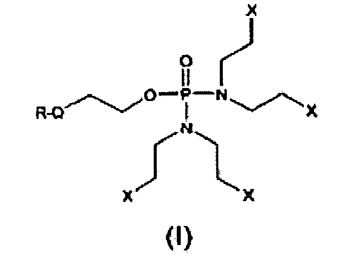 dans laquelle :
X représente un atome d'halogène ;
Q représente S ; et
R représente un atome d'hydrogène, un groupe alkyle en C₁ à C₆, alkyle en C₁ à C₆ substitué, aryle en C₆ à C₁₄, aryle en C₆ à C₁₄ substitué, hétéroaryle en C₅ à C₁₄ ou hétéroaryle en C₅ à C₁₄ substitué, ou bien représente un groupe R'CO-, R'NHCO-, R'SO₂ ou R'NHSO₂-, dans lequel R' représente un atome d'hydrogène, un groupe alkyle en C₁ à C₆, alkyle en C₁ à C₆ substitué, aryle en C₆ à C₁₄, aryle en C₆ à C₁₄ substitué, hétéroaryle en C₅ à C₁₄ ou hétéroaryle en C₅ à C₁₄ substitué ; ledit groupe alkyle en C₁ à C₆ substitué étant mono-, di- ou trisubstitué avec un substituant aryle en C₆ à C₁₄, aryle en C₆ à C₁₉ à substituant R¹, hétéroaryle en C₅ à C₁₄, nitro, cyano, halogéno, -OR¹, -SR¹, -C(O)R¹, -OC(O)R¹, -C(O)OR¹, -NR¹₂, -SO₂OR¹, -OSO₂R¹, -SO₂NR¹₂, -NR¹SO₂R¹, -CONR¹₂ ou -NR¹C(O)R¹, dans lequel chaque groupe R¹ représente, indépendamment, un atome d'hydrogène, un groupe alkyle en C₁ à C₆, alkyle en C₁ à C₆ à substituant R², aryle en C₆ à C₁₄, aryle en C₆ à C₁₄ à substituant R², hétéroaryle en C₅ à C₁₄, (hétéroaryle en C₅ à C₁₄) (alkyle en C₁ à C₆) , (aryle en C₆ à C₁₄) (alkyle en C₁ à C₆) à substituant R² ou (aryle en C₆ à C₁₄) (alkyle en C₁ à C₆) et chaque groupe R² représente, indépendamment, un groupe hydroxy, halogéno, alkyle en C₁ à C₆, cyano, thio, nitro, alkyle en C₁ à C₆, halogénalkyle en C₁ à C₆ ou amino ;
et ses sels pharmaceutiquement acceptables.

13. Composé suivant la revendication 12, dans lequel X représente un atome de chlore ou de brome.

14. Composé suivant la revendication 13, dans lequel X représente un atome de chlore.

15. Composé suivant l'une quelconque des revendications 12 à 14, dans lequel R représente un atome d'hydrogène, un groupe alkyle en C₁ à C₆, alkyle à C₁ à C₆ substitué, aryle en C₆ à C₁₄, aryle en C₆ à C₁₄ substitué, hétéroaryle en C₅ à C₁₄ ou hétéroaryle en C₅ à C₁₄ substitué.

16. Composé suivant la revendication 14, dans lequel R représente H.

17. Composition pharmaceutique comprenant un composé de l'une quelconque des revendications 12 à 16.

18. Composition pharmaceutique suivant la revendication 17 pour le traitement de tumeurs, en particulier d'un cancer, chez un mammifère.

19. Composé suivant l'une quelconque des revendications 12 à 16, destiné à être utilisé dans le traitement de tumeurs, en particulier d'un cancer, chez un mammifère.

20. Procédé pour la préparation d'un composé de formule (I) suivant la revendication 12, le procédé comprenant
(a) la réaction d'un composé de formule (2) 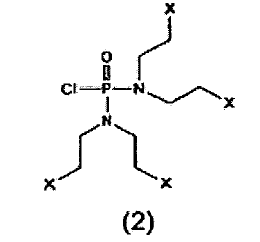 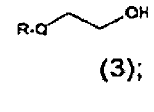 or
(b) la transformation de substituants d'un composé de formule I d'une manière en elle-même connue ; ou
(c) la réaction de la base libre d'un composé de formule I avec un acide pour obtenir un sel d'addition d'acide pharmaceutiquement acceptable, ou
(d) la réaction d'un sel d'addition d'acide d'un composé de formule I avec une base pour former la base libre correspondante, ou
(e) la conversion d'un sel d'un composé de formule I en un autre sel pharmaceutiquement acceptable d'un composé de formule I ; ou
(f) la résolution d'un mélange racémique en n'importe quelles proportions d'un composé de formule I pour obtenir un de ses stéréoisomères.
